# EUROPEAN PATENT APPLICATION

(11) **EP 4 741 829 A1**
(43) Date of publication of application: **13.05.2026**
(21) Application number: 24835857.4
(22) Date of filing: 11.06.2024
(51) Int. Cl.: G01N 35/00

(54) **AUTOMATIC ANALYSIS DEVICE AND ANALYSIS RESULTS DISPLAY METHOD**

(30) Priority: 06.07.2023 JP 2023111311
(71) Applicant: HITACHI HIGH-TECH CORPORATION, Tokyo 105-6409 (JP)
(72) Inventor: SHIBA, Kazuaki, Tokyo 105-6409 (JP); CHIKAHISA, Masaki, Tokyo 105-6409 (JP)
(74) Representative: MERH-IP Matias Erny Reichl Hoffmann Patentanwälte PartG mbB
(86) International application number: PCT/JP2024/021252
(87) International publication number: WO 2025/009340

(57) **Abstract**

The purpose of the present invention is to provide an automatic analysis device with high processing capability by enabling updating of an analysis parameter without going into a standby state. To this end, the present invention is an automatic analysis device that comprises: an analysis module that analyzes a specimen; a storage unit that stores analysis results of the analysis module; a display unit that displays the analysis results stored in the storage unit; and a control unit that controls the analysis module, the storage unit, and the display unit. The storage unit stores, in association with each other, the analysis results and an analysis parameter applied to the analysis. The display unit displays the version of the analysis parameter for each of the analysis results.

## Description

### Technical Field

The present invention relates to an automatic analysis device and an analysis result display method.

### Background Art

An automatic analysis device that automatically performs quantification and qualitative analysis of a biological sample (hereinafter, referred to as a specimen) such as blood or urine performs an analysis operation according to an analysis parameter set in advance corresponding to a predetermined item when there is an analysis request for the item. For example, PTL 1 describes an automatic analysis device that classifies analysis parameters into fixed parameters and variable parameters in order to set the analysis parameters reflecting characteristics of different reagents for each reagent lot, and controls an analysis operation by these parameters. Generally, the analysis parameter is version-managed, and the version thereof is updated according to an inspection target and an inspection status.

### Citation List

### Patent Literature

PTL 1: JP2012-107985A

### Summary of Invention

### Technical Problem

In the automatic analysis device of the related art including the technique of PTL 1, when the analysis parameter is updated during the analysis operation or the like, the analysis result and the parameter applied to the analysis cannot be associated with each other, and thus a timing of updating the analysis parameter is restricted when the device is in a standby state. Therefore, when a user wants to update the analysis parameter, the user needs to set the device to the standby state first, and a waiting time is required until the device is set to an analyzable state again, which may lead to a decrease in a processing capability.

An object of the present invention is to provide an automatic analysis device with a high processing capability by enabling update of an analysis parameter without setting a standby state.

### Solution to Problem

In order to achieve the above object, according to the invention, there is provided an automatic analysis device including an analysis module configured to analyze a specimen, a storage unit configured to store an analysis result in the analysis module, a display unit configured to display the analysis result stored in the storage unit, and a control unit configured to control the analysis module, the storage unit, and the display unit. The storage unit accumulates the analysis result and an analysis parameter applied to analysis in association with each other, and the display unit displays a version of the analysis parameter for each analysis result.

### Advantageous Effects of Invention

According to the present invention, it is possible to provide an automatic analysis device having a high processing capability by enabling update of an analysis parameter without setting a standby state.

### Brief Description of Drawings

[FIG. 1] FIG. 1 is a front view illustrating an appearance of an overall configuration of an automatic analysis device.
[FIG. 2] FIG. 2 is a top view illustrating an outline of the overall configuration of the automatic analysis device.
[FIG. 3] FIG. 3 is a flowchart illustrating an overall analysis operation of the automatic analysis device.
[FIG. 4] FIG. 4 is a flowchart illustrating analysis parameter update processing in an automatic analysis device of the related art.
[FIG. 5] FIG. 5 is a diagram illustrating an example of an analysis result confirmation screen and a linked database stored in a storage unit in an automatic analysis device according to Embodiment 1.
[FIG. 6] FIG. 6 is a diagram illustrating an example of an analysis parameter setting screen.
[FIG. 7] FIG. 7 is a diagram illustrating an example of an analysis parameter update screen.
[FIG. 8] FIG. 8 is a flowchart illustrating analysis parameter update processing in the automatic analysis device according to Embodiment 1.
[FIG. 9] FIG. 9 is a diagram illustrating an example of a version-specific parameter information screen and an integrated database stored in a storage unit in an automatic analysis device according to Embodiment 2.
[FIG. 10] FIG. 10 is a diagram illustrating an example of an application timing setting screen of an updated analysis parameter.
[FIG. 11] FIG. 11 is a diagram illustrating an example of a setting screen for designating an analysis item to be automatically updated.
[FIG. 12] FIG. 12 is a flowchart illustrating analysis parameter update processing in an automatic analysis device according to Embodiment 3.

### Description of Embodiments

Hereinafter, embodiments of the invention will be described with reference to the drawings.

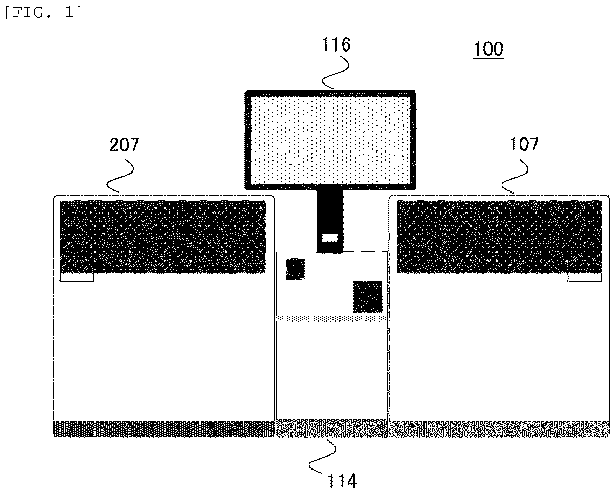

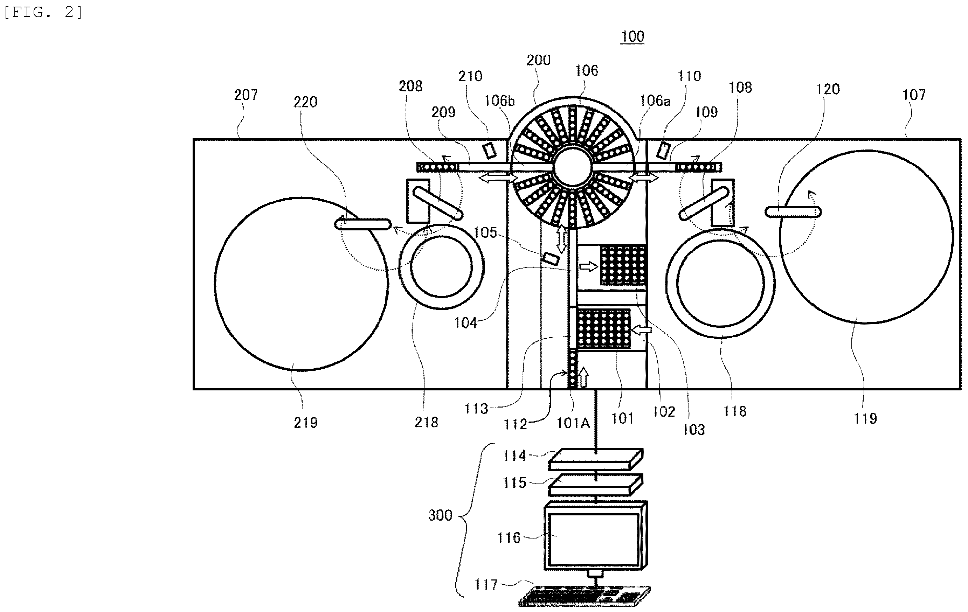

First, a configuration of an automatic analysis device will be described with reference to FIGS. 1 and 2. FIG. 1 is a front view illustrating an appearance of an overall configuration of the automatic analysis device, and FIG. 2 is a top view illustrating an outline of the overall configuration of the automatic analysis device. The automatic analysis device 100 according to the present embodiment includes a sampler module 200, analysis modules 107, 207, and a control device 300.

The sampler module 200 is a module that transports a specimen rack loaded into the automatic analysis device 100 to and from the analysis modules 107, 207. The sampler module 200 includes a transport line 104, an emergency specimen rack loading unit 112, a specimen rack supply unit 102, a specimen rack storage unit 103, an emergency specimen rack waiting area 113, a specimen identification device 105, a rack rotor 106, and the like.

In the specimen rack, one or more specimen containers containing a specimen to be subjected to qualitative and quantitative analysis in the analysis modules 107, 207 are mounted. The specimen rack includes at least a normal specimen rack 101 and an emergency specimen rack 101A. The normal specimen rack 101 is a specimen rack on which specimen containers containing specimens (normal specimens) to be analyzed with normal priority are mounted. The emergency specimen rack 101A is a specimen rack on which specimen containers containing specimens (emergency specimens) having a higher priority of analysis than the normal specimen are mounted.

Here, details of each configuration included in the sampler module 200 will be described. First, the transport line 104 reciprocally transports the specimen rack 101 and the emergency specimen rack 101A, and is, for example, a belt conveyor type transport mechanism. The transport line 104 may use a mechanism that transports a protruding structure driven along the transport line 104 by fitting the protruding structure into a recess provided in advance in the specimen rack.

The emergency specimen rack loading unit 112 is provided adjacent to the transport line 104, and is an area for loading the emergency specimen rack 101A. The specimen rack supply unit 102 is provided adjacent to the transport line 104 closer to one end side of the transport line 104 than the emergency specimen rack loading unit 112, and is an area for supplying the normal specimen rack 101. The specimen rack storage unit 103 is provided adjacent to the transport line 104 closer to the one end side of the transport line 104 than the specimen rack supply unit 102, and is an area for storing the specimen rack 101. The emergency specimen rack waiting area 113 is provided on the transport line 104 closer to the other end side of the transport line 104 than the specimen rack storage unit 103, and is an area for temporarily waiting the emergency specimen rack 101A. The specimen identification device 105 is a mechanism that reads an identification medium (not illustrated) such as an RFID or a barcode provided in the specimen rack 101, the emergency specimen rack 101A, and the specimen container, and identifies analysis request information on the transported specimen.

The rack rotor 106 is disposed at one end of the transport line 104. The rack rotor 106 has slots 106a, 106b on which the specimen rack 101 and the like can be mounted, and is a mechanism for transferring the specimen rack 101 and the like between one end of the transport line 104 and one end of each of dispensing lines 109, 209 of the analysis modules 107, 207. The rack rotor 106 is configured to rotate clockwise and counterclockwise. A rotation operation of the rack rotor 106 is controlled such that start processing starts in an order in which the specimen rack 101 is loaded, or the processing starts before the normal specimen rack 101 when the emergency specimen rack 101A having a high priority is loaded.

Each of the analysis modules 107, 207 is a unit that performs qualitative and quantitative analysis by performing sampling (dispensing) on a specimen contained in the specimen container mounted on the specimen rack. The analysis modules 107, 207 include the dispensing lines 109, 209, specimen identification devices 110, 210, reaction disks 118, 218, specimen dispensing mechanisms 108, 208, reagent disks 119, 219, reagent dispensing mechanisms 120, 220, measurement units (not illustrated), and the like, respectively.

The dispensing lines 109, 209 include a transport mechanism that can perform a reciprocating operation of pulling in the specimen rack from the sampler module 200 to the analysis modules 107, 207 and delivering the specimen rack from the analysis modules 107, 207 to the sampler module 200.

FIG. 2 illustrates an example of a belt conveyor type transport mechanism as the dispensing lines 109, 209, but instead of this, a mechanism that transports a protruding structure driven along the dispensing lines 109, 209 by fitting the protruding structure into a recess provided in advance in the specimen rack may be used.

The specimen identification devices 110, 210 are each a mechanism that reads an identification medium (not illustrated) such as an RFID or a barcode provided in the specimen rack 101, the emergency specimen rack 101A, and the specimen container, and identifies analysis request information on the transported specimen. The specimen dispensing mechanisms 108, 208 are mechanisms that dispense specimens from specimen containers of specimen racks, transported to dispensing positions on the dispensing lines 109, 209, into reaction containers of the reaction disks 118, 218. The reagent dispensing mechanisms 120, 220 are mechanisms that dispense reagents contained in the reagent containers of the reagent disks 119, 219 into the reaction containers of the reaction disks 118, 218. The measurement unit is a mechanism that performs qualitative and quantitative analysis by measuring a mixed solution (reaction solution) of a specimen and a reagent dispensed into the reaction container.

The purpose of the test (test item) and a processing capability of each analysis module are different, and for example, the analysis module 107 is assumed to be a biochemical test unit, and the analysis module 207 is assumed to be an immunological test unit. In addition, a unit for electrolyte concentration measurement can be provided in the analysis module 107, or a unit for blood coagulation analysis or the like can be appropriately arranged in each module according to a specification environment. The number of analysis modules is two in the present embodiment, but may be three or more.

The control device 300 is a computer that controls an overall operation of the automatic analysis device 100 including the respective configurations of the analysis modules 107, 207 and the sampler modules 200, and includes a display unit 116, an input unit 117, a storage unit 115, and the like in addition to a control unit 114. The control unit 114 includes a CPU, a memory, and the like. The display unit 116 is a display device such as a liquid crystal display that displays various setting screens including analysis parameter settings, an analysis item request screen, an analysis result confirmation screen, maintenance information, and the like. The input unit 117 is, for example, a keyboard or a mouse, and receives an input from a user. The storage unit 115 stores information such as an analysis result in each analysis module and contents of various settings.

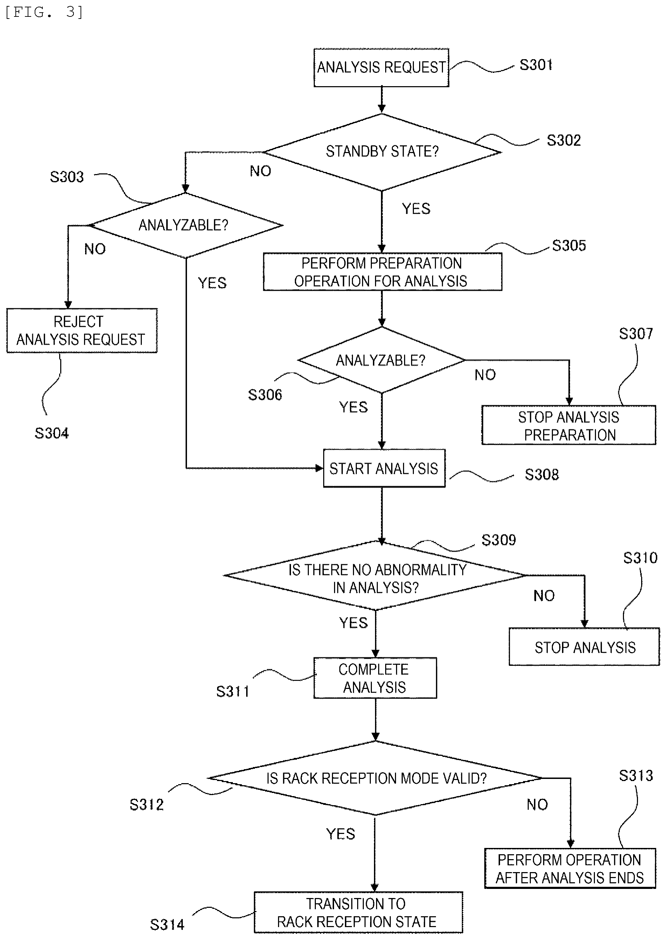

Next, a flow of an overall analysis operation in the automatic analysis device having the above configuration will be described with reference to FIG. 3. FIG. 3 is a flowchart illustrating the overall analysis operation of the automatic analysis device.

When the control unit 114 receives an analysis request from the user via the input unit 117 (step S301), the control unit 114 determines whether the automatic analysis device is in a standby state (step S302). When it is determined that the automatic analysis device is in the standby state, the control unit 114 performs a preparation operation for analysis such as a reagent registration operation or a sensor confirmation operation (step S305).

On the other hand, when it is determined in step S302 that the automatic analysis device is not in the standby state, the control unit 114 determines whether the state is an analyzable state (step S303). When the analysis operation of another specimen is in progress or when the rack is in a rack reception state, it is determined that the state is in the analyzable state, and analysis of the specimen requested to be analyzed is started (step S308). However, when the device is in a startup operation or in maintenance execution, it is determined that dispensing is impossible, and the analysis request is rejected (step S304). The rack reception state is a state in which transition to the analysis operation can be made in a shorter time than in the standby state. In the rack reception state, for example, the dispensing mechanism stops similarly to the standby state, but the reaction disk does not stop unlike the standby state, and thus, when the analysis request is received from the user, the preparation operation for analysis can be omitted and the analysis can be started.

While the preparation operation for analysis is being performed in step S305, the control unit 114 also checks a state of the reagent and a state of the sensor, and continuously determines whether the reagent and the sensor are in the analyzable state (step S306). When a remaining amount of the reagent is insufficient, it is determined that the reagent is in an unanalyzable state, and the preparation for analysis is stopped (step S307). On the other hand, when the preparation operation for analysis is completed in the analyzable state, the control unit 114 starts analysis (step S308).

Even during the analysis, the control unit 114 continuously determines whether there is no abnormality in the analysis, such as confirming the remaining amount of the reagent (step S309), and stops the analysis when it is determined that there is an abnormality (step S310). When the analysis is completed without abnormality (step S311), the control unit 114 determines whether the setting of a rack reception mode is valid (step S312). When it is determined that the rack reception mode is valid, the automatic analysis device transitions to the rack reception state (step S314), and continues the rack reception state for a time designated in advance by the user. On the other hand, when it is determined that the rack reception mode is invalid, the automatic analysis device performs an operation after the analysis ends (step S313) and then transitions to the standby state.

During a period from step S305 to step S314 in FIG. 3, the user can forcibly stop the analysis operation and the rack reception state and transition the automatic analysis device to the standby state by operating a stop button displayed on the display unit with the input unit. However, when the automatic analysis device performs the analysis again after transitioning to the standby state, the automatic analysis device needs to perform the operations in step S305 and subsequent steps again.

Here, each analysis module of the automatic analysis device performs the analysis according to the analysis parameter set in advance by the control device 300. It is desirable that this analysis parameter not only needs to be updated (corrected) when there is a defect, but also is updated to a more suitable parameter according to an analysis situation. Hereinafter, specific processing from when the control device 300 receives an instruction to update the analysis parameter from the user to when the control device 300 applies the updated analysis parameter to the analysis will be described. First, processing in an automatic analysis device of the related art will be described as a comparative example, and then processing in the automatic analysis device according to the embodiment will be described.

### (Comparative Example)

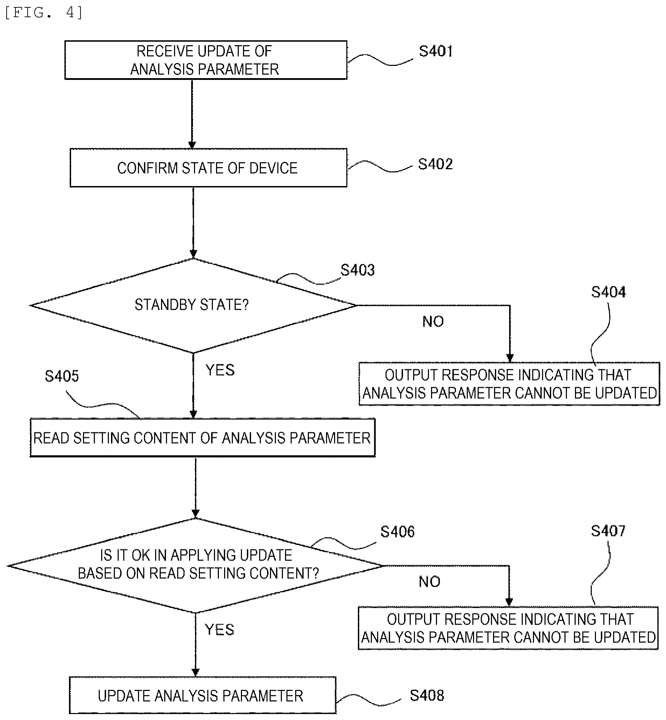

FIG. 4 is a flowchart illustrating analysis parameter update processing in the automatic analysis device of the related art.

When the control unit receives the instruction to update the analysis parameter (step S401), the control unit confirms the state of the automatic analysis device (step S402). Next, the control unit determines whether the state of the automatic analysis device is the standby state (step S403). When it is determined that the state is not the standby state, the control unit causes the display unit to output a response indicating that the analysis parameter cannot be updated (step S404), and interrupts the update processing.

When it is determined in step S403 that the state is the standby state, the control unit reads a setting content of the analysis parameter for which the update instruction is received (step S405), and determines whether it is OK in applying the update based on the read setting content (step S406). When it is determined that it is not OK, for example, when the reagent dispensing amount is not within a measurable range, the control unit causes the display unit to output a response indicating that the analysis parameter cannot be updated (step S407), and interrupts the update processing.

When it is determined in step S406 that it is OK, the control unit immediately applies the updated analysis parameter (step S408), and then performs processing of calibration, quality control, and general analysis. The order of step S403 and step S406 may be reversed.

As described above, the automatic analysis device according to the comparative example cannot update the analysis parameter unless in the standby state. Therefore, when the automatic analysis device is during the analysis operation or in the rack reception state at the time when the user desires to update the analysis parameter, the user needs to forcibly transition the automatic analysis device to the standby state by operating the stop button. However, when the automatic analysis device performs the analysis again after transitioning to the standby state, as described above, the automatic analysis device needs to perform the preparation operation and the like in step S305, and it takes time to start the analysis again.

Here, some analysis parameters have a small influence on the analysis result even when updated, and in such a case, it is considered that the automatic analysis device does not necessarily need to immediately transition to the standby state even during the analysis operation. However, in the automatic analysis device according to the comparative example, when the analysis parameter can be updated even during the analysis operation, the user cannot grasp association between the analysis result and the version of the analysis parameter applied to the analysis. This is because, in the automatic analysis device according to the comparative example, information of the analysis parameter is also recorded in the storage unit, but the information of the analysis parameter is not displayed on the analysis result confirmation screen. Although it is possible to roughly estimate which version of the analysis parameter is applied to which analysis with reference to a date and time when the analysis parameter is updated, it is difficult to determine them definitively. In particular, when the analysis parameter is updated during the analysis operation of the automatic analysis device, it is also difficult to estimate the version of the analysis parameter applied to the analysis.

### Embodiment 1

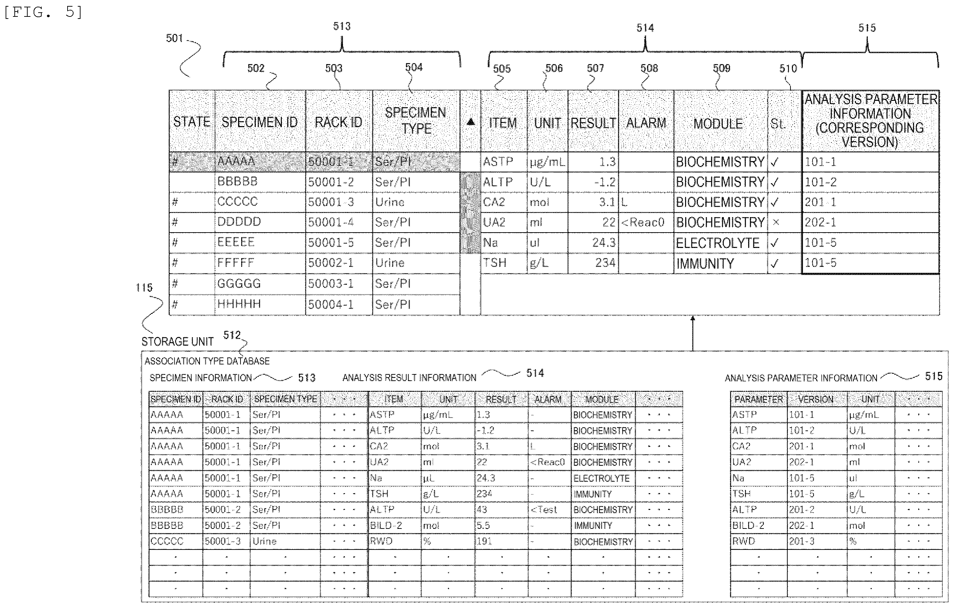

An automatic analysis device according to Embodiment 1 will be described with reference to FIGS. 5 to 8.

FIG. 5 is a diagram illustrating an example of an analysis result confirmation screen and a linked database stored in a storage unit in the automatic analysis device according to Embodiment 1. The automatic analysis device according to Embodiment 1 causes the display unit to display an analysis result screen 501 (the analysis result confirmation screen) based on the information accumulated in the association type database 512 in the storage unit 115.

When the analysis result is output, related information is cumulatively recorded in the association type database 512 regardless of whether the analysis result is successful or unsuccessful. In the association type database 512, specimen information 513 and analysis result information 514 are accumulated, and analysis parameter information 515 (for example, the version of the analysis parameter) is also accumulated in association with these pieces of information. The specimen information 513 includes, for example, a specimen ID 502, a rack ID 503, and a specimen type 504. The analysis result information 514 includes, for example, an analysis item name 505, a unit 506, a result 507 (measurement value), a data alarm result 508, a use analysis module 509, and an analysis status 510. Even if the user deletes the analysis result information 514 and the analysis parameter information 515, the analysis result information 514 and the analysis parameter information 515 are not deleted from the association type database 512 and are managed as accumulatively recorded information.

The information read from the association type database 512 is output to the analysis result screen 501. In the analysis result screen 501, a list of specimens analyzed in the past is displayed in a column of the specimen information 513. When a specific specimen is selected by the user from the column of specimen information 513, the display unit displays not only analysis result information 514 corresponding to the specimen but also analysis parameter information 515 applied to the analysis for each analysis result. FIG. 5 illustrates a state in which AAAAA is selected as the specimen ID, and all analysis results performed on the specimen and the version of the analysis parameter in each analysis are displayed.

As described above, since the version of the analysis parameter is displayed for each analysis result on the analysis result screen 501, even when the analysis parameter is updated during the analysis operation, the user can grasp the association between the analysis result and the analysis parameter applied to the analysis.

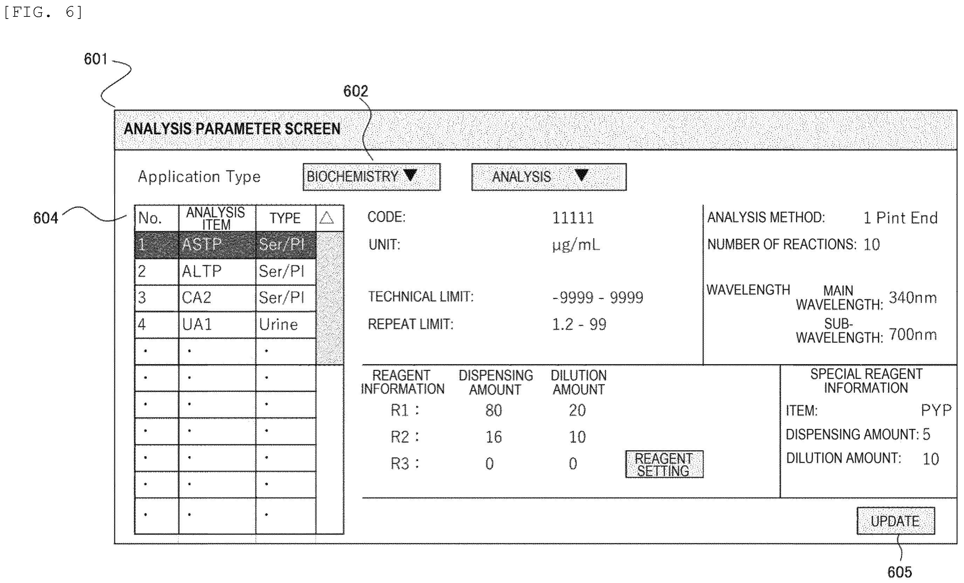

FIG. 6 is a diagram illustrating an example of an analysis parameter setting screen. On an analysis parameter screen 601, it is possible to confirm the setting content of the analysis parameter and update the setting content of the analysis parameter. When the user selects a module type using a combo box 602 for analysis module type selection, the display unit displays all analysis items corresponding to the selected analysis module in an analysis item list 604. Further, when a predetermined analysis item is selected by the user from the analysis item list 604, the display unit displays detailed information of the analysis parameter currently set for the analysis item. The detailed information of the analysis parameter includes, for example, basic information such as a code number, a unit, and a technical limit, and reagent-related information such as a reagent dispensing amount, a dilution amount, and a special reagent to be used at the time of analysis. When the user desires to update the analysis parameter, an update button 605 is operated by the input unit.

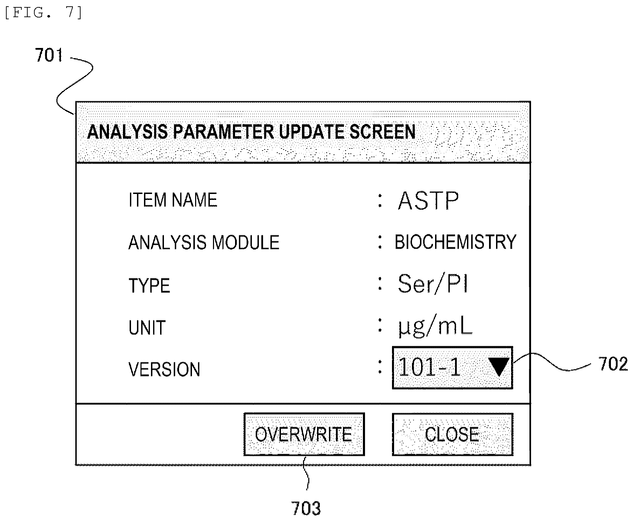

When the update button 605 is operated on the analysis parameter screen 601 of FIG. 6, the display unit displays an analysis parameter update screen 701 illustrated in FIG. 7. FIG. 7 is a diagram illustrating an example of the analysis parameter update screen. When the user selects an application version in a combo box 702 for an analysis parameter version and operates an overwrite button 703, the analysis parameter is updated.

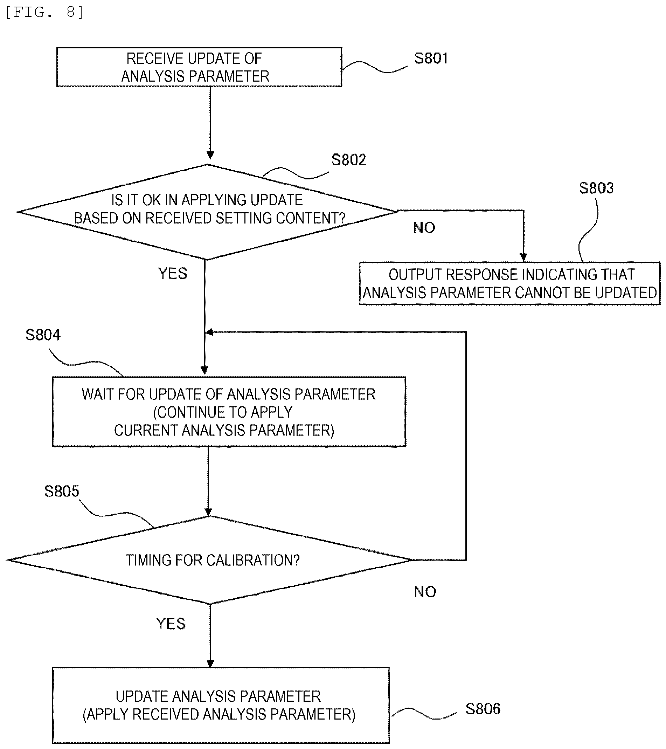

FIG. 8 is a flowchart illustrating the analysis parameter update processing in the automatic analysis device according to Embodiment 1.

When the control unit receives the instruction to update the analysis parameter through the screens illustrated in FIGS. 6 and 7 (step S801), the control unit determines whether it is OK in applying the update based on the received setting content (step S802). When it is determined that it is not OK, for example, when the reagent dispensing amount is not within the measurable range, the control unit causes the display unit to output the response indicating that the analysis parameter cannot be updated (step S803), and interrupts the update processing.

When it is determined in step S802 that it is OK, the control unit does not immediately apply the updated analysis parameter, continues to apply the current analysis parameter, and waits for the update of the analysis parameter (step S804). Next, the control unit determines whether the state of the automatic analysis device reaches a timing for calibration (step S805), and continues to apply the current analysis parameter until the timing for calibration is reached. At the timing for calibration, the control unit applies the updated analysis parameter based on the received setting content (step S806).

As described above, in Embodiment 1, even when the automatic analysis device is not in the standby state, the user can instruct to update the analysis parameter. That is, since it is not necessary to immediately transition to the standby state, it is possible to eliminate the waiting time until the analyzable state is set again and improve the processing capability. Further, in Embodiment 1, when there is an update instruction from the user during the analysis operation or in the rack reception state of the automatic analysis device, the analysis operation or the like can be continued until a timing for the next calibration while applying the analysis parameter of the version before the update. In particular, in the case of the analysis parameter having a small influence on the analysis result, a certain degree of accuracy can be ensured even for the analysis result based on the analysis parameter before the update.

### Embodiment 2

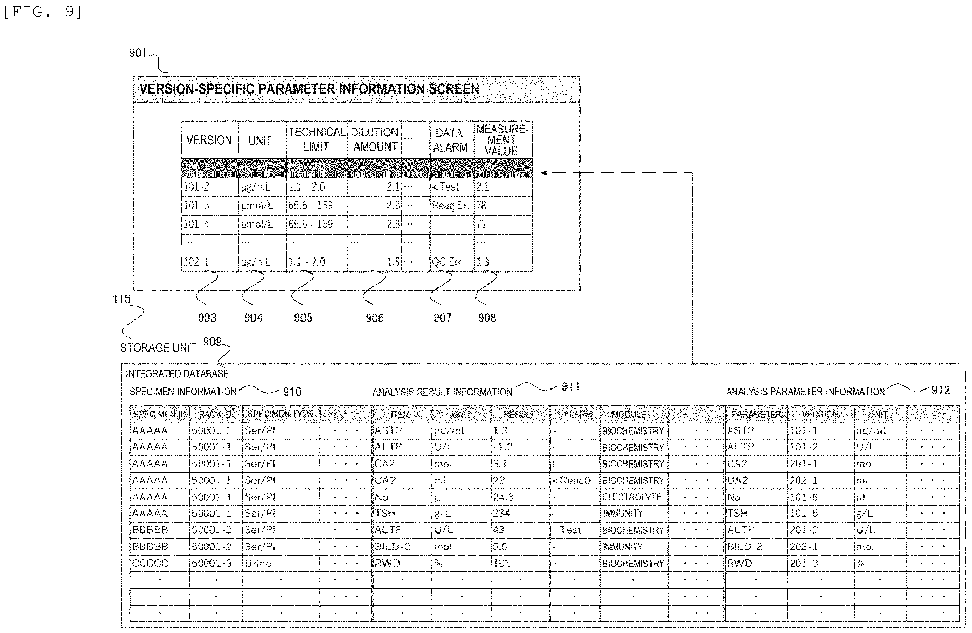

An automatic analysis device according to Embodiment 2 will be described with reference to FIG. 9. The following description focuses on differences from Embodiment 1.

FIG. 9 is a diagram illustrating an example of a version-specific parameter information screen and an integrated database stored in a storage unit in the automatic analysis device according to Embodiment 2. The automatic analysis device according to Embodiment 2 displays a version-specific parameter information screen 901 on the display unit based on the information accumulated in an integrated database 909 in the storage unit 115.

When the analysis result is output, similar to the association type database of Embodiment 1, related information is cumulatively recorded in the integrated database 909 regardless of whether the analysis result is successful or unsuccessful. Specimen information 910, analysis result information 911, and analysis parameter information 912 are accumulated in an integrated form in the integrated database 909.

On the version-specific parameter information screen 901, the setting content of the analysis parameter and the analysis result are displayed side by side for each version as information read from the integrated database 909. The setting content of the analysis parameter includes, for example, a version 903, a unit 904, a technical limit 905, and a dilution amount 906. The analysis result includes, for example, a data alarm 907 and a measurement value 908.

Here, not only the setting content of the analysis parameter of the actually applied version and the actually obtained analysis result but also a setting content of an analysis parameter of another version and an analysis result predicted when the analysis parameter is applied are displayed on the version-specific parameter information screen 901. The prediction of the analysis result is performed by the control unit using an actual analysis result accumulated in the integrated database 909. The predicted analysis result may be accumulated in the integrated database 909 in association with the actual analysis result. FIG. 9 illustrates a state in which an analysis item of ASTP for the specimen having the specimen ID of AAAAA is selected, and not only data of an actually applied version "101-1" but also data of other versions are displayed on the version-specific parameter information screen 901.

As described above, in Embodiment 2, since the case where the version is updated and the case where the version is not updated can be compared, the user can visually confirm the effect of version update. In addition, since a predicted value when another version that is not actually applied is applied is also displayed, when an actual analysis result is unexpected, the predicted value serves as a criterion for finding a valid version.

### Embodiment 3

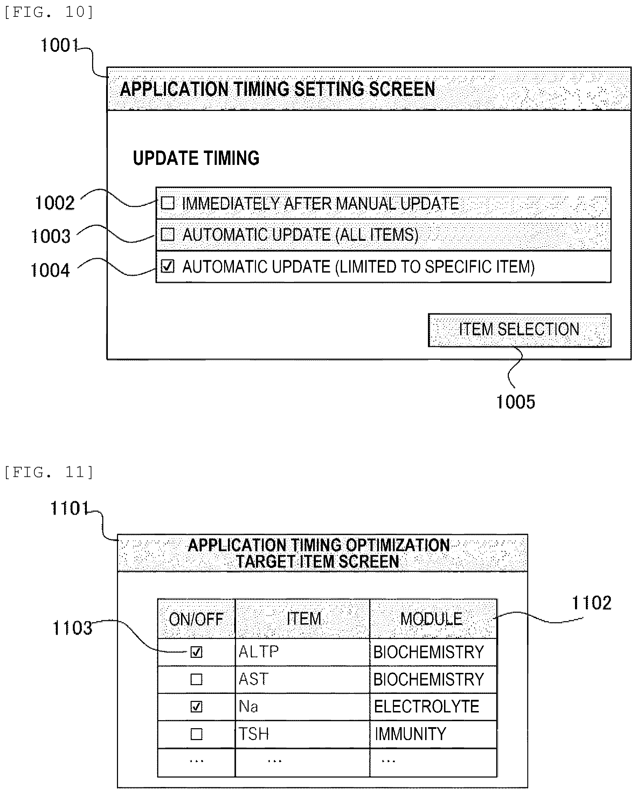

An automatic analysis device according to Embodiment 3 will be described with reference to FIGS. 10 to 12. The following description focuses on differences from Embodiment 1.

In Embodiment 1 described above, even when the instruction to update the analysis parameter is received, the application of the current analysis parameter is continued, and the updated analysis parameter is applied at the timing for calibration. However, in the case of the analysis parameter having a large influence on the analysis result, the accuracy of the analysis result cannot be ensured unless the update of the analysis parameter and the calibration are immediately performed.

In particular, in a case of an analysis item of an immunological test, when the instruction to update the analysis parameter is received from the user, it is desirable that the control unit immediately applies the updated analysis parameter and does not perform general analysis or quality control regarding the analysis item until next calibration is performed. Therefore, in Embodiment 3, the user can select an application timing of the updated analysis parameter by the input unit.

FIG. 10 is a diagram illustrating an example of an application timing setting screen of the updated analysis parameter.

When a radio button 1002 for immediately after manual update is selected on the application timing setting screen 1001, when the instruction to update the analysis parameter is received, a mode in which the update is immediately applied is set. When a radio button 1003 for automatic update (all items) is selected on the application timing setting screen 1001, a mode is set in which the update is applied after the next calibration regardless of which the instruction to update the analysis parameter is received.

When an item selection button 1005 is operated in a state in which a radio button 1004 for automatic update (a specific item) is selected on the application timing setting screen 1001, the display unit displays an application timing optimization target item screen 1101 illustrated in FIG. 11. FIG. 11 is a diagram illustrating an example of a setting screen for designating an analysis item to be automatically updated. In an analysis item list 1102, the analysis item in which an application switching check box 1103 is enabled is automatically updated, that is, a mode in which the update is applied after the next calibration even when the instruction to update the analysis parameter is received is set.

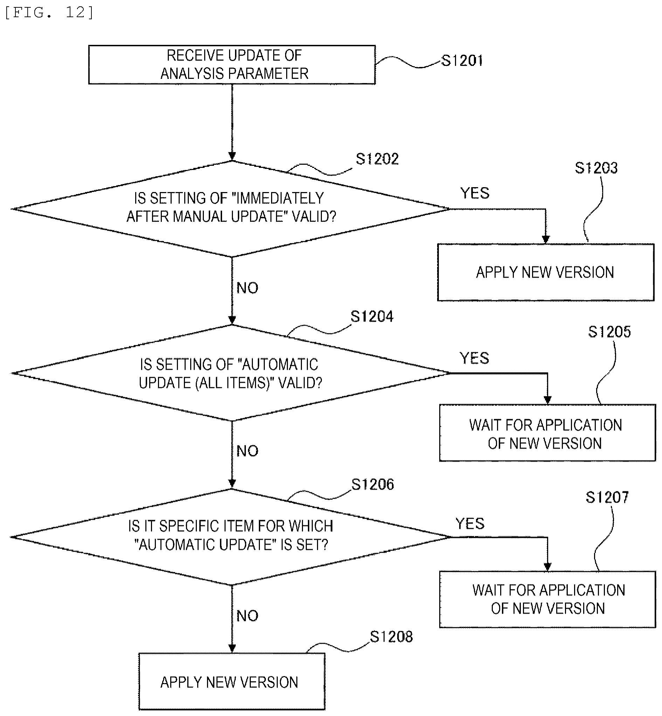

FIG. 12 is a flowchart illustrating the analysis parameter update processing in the automatic analysis device according to Embodiment 3. Here, a case where the automatic analysis device receives the instruction to update the analysis parameter during the analysis operation or in the rack reception state will be described.

When the control unit receives the instruction to update the analysis parameter (step S1201), the control unit determines whether the setting of "immediately after manual update" is valid (step S1202). When it is determined that the setting is valid, the control unit immediately applies the updated new version from the immediately subsequent analysis (step S1203).

When it is determined in step S1202 that the setting is invalid, the control unit determines whether the setting of "automatic update (all items)" is valid (step S1204). When it is determined that the setting is valid, the control unit continues the analysis operation or the rack reception state while holding the version of the analysis parameter at the present time, and applies the updated new version at the timing of the next calibration (step S1205).

When it is determined in step S1204 that the setting is invalid, the control unit determines whether the item is an optimization target item for which "automatic update" is set (step S1206). When it is determined that the item is the optimization target item, the control unit holds the version of the analysis parameter at the present time and applies the updated new version at the timing of the next calibration (step S1207).

When it is determined in step S1206 that the item is not the optimization target item, the control unit immediately applies the updated new version (step S1208).

As described above, in Embodiment 3, since the application timing can be changed according to the analysis item, it is possible to ensure the accuracy of the analysis result and improve the processing capability.

The invention is not limited to the embodiments described above, and includes various modifications. For example, the embodiments described above have been described in detail to facilitate understanding of the invention, and the invention is not necessarily limited to those including all the configurations described above. A part of a configuration in one embodiment can be replaced with a configuration in another embodiment, and a configuration in one embodiment can also be added to a configuration in another embodiment. Further, a part of a configuration in each embodiment may also be added to, deleted from, or replaced with another configuration.

### Reference Signs List

100: automatic analysis device
101: specimen rack
101A: emergency specimen rack
102: specimen rack supply unit
103: specimen rack storage unit
104: transport line
105: specimen identification device
106: rack rotor
106a, 106b: slot
107, 207: analysis module
108, 208: specimen dispensing mechanism
109, 209: dispensing line
110, 210: specimen identification device
112: emergency specimen rack loading unit
113: emergency specimen rack waiting area
114: control unit
115: storage unit
116: display unit
117: input unit
118, 218: reaction disk
119, 219: reagent disk
120, 220: reagent dispensing mechanism
200: sampler module
300: control device
501: analysis result screen
502: specimen ID
503: rack ID
504: specimen type
505: analysis item name
506: unit
507: result
508: data alarm result
509: use analysis module
510: analysis status
512: association type database
513: specimen information
514: analysis result information
515: analysis parameter information
601: analysis parameter screen
602: combo box for analysis module type selection
604: analysis item list
605: update button
701: analysis parameter update screen
702: combo box for analysis parameter version
703: overwrite button
901: version-specific parameter information screen
903: version
904: unit
905: technical limit
906: dilution amount
907: data alarm
908: measurement value
909: integrated database
910: specimen information
911: analysis result information
912: analysis parameter information
1001: application timing setting screen
1002: radio button for immediately after manual update
1003: radio button for automatic update (all items)
1004: radio button for automatic update (specific item)
1005: item selection button
1101: application timing optimization target item screen
1102: analysis item list
1103: application switching check box

## Claims

1. An automatic analysis device comprising:
an analysis module configured to analyze a specimen;
a storage unit configured to store an analysis result in the analysis module;
a display unit configured to display the analysis result stored in the storage unit; and
a control unit configured to control the analysis module, the storage unit, and the display unit, wherein
the storage unit accumulates the analysis result and an analysis parameter applied to analysis in association with each other, and
the display unit displays a version of the analysis parameter for each analysis result.

2. The automatic analysis device according to claim 1, wherein
even when the update of the analysis parameter is received during an analysis operation or in a rack reception state, the control unit continues the analysis operation or the rack reception state while applying the analysis parameter before the update until next calibration is performed.

3. The automatic analysis device according to claim 1, wherein
the control unit predicts an analysis result when an analysis parameter of a version different from an actually applied version is temporarily applied, and
the storage unit accumulates the analysis result predicted by the control unit and an actual analysis result in association with each other.

4. The automatic analysis device according to claim 3, wherein
when a predetermined analysis item for a predetermined specimen is selected,
the display unit displays a setting content of the analysis parameter and an analysis result side by side for each version.

5. The automatic analysis device according to claim 1, further comprising
an input unit configured to allow a setting content of the analysis parameter to be input therein and allow an application timing of an updated analysis parameter to be input therein, wherein
in a case where the application timing is set to immediate, when the update of the analysis parameter is received by the input unit during an analysis operation or in a rack reception state, the control unit applies the updated analysis parameter to immediately subsequent analysis, and
in a case where the application timing is set to calibration, when the update of the analysis parameter is received by the input unit during an analysis operation or in a rack reception state, the control unit applies the updated analysis parameter to analysis starting from next calibration.

6. The automatic analysis device according to claim 5, wherein
the application timing is capable of being set for each analysis item.

7. An analysis result display method for displaying a result of analyzing a specimen by an automatic analysis device, the display method comprising:
displaying, for each analysis result, a version of an analysis parameter applied to analysis.
